# EUROPEAN PATENT APPLICATION

(11) **EP 3 572 399 A1**
(43) Date of publication of application: **27.11.2019**
(21) Application number: 18741904.9
(22) Date of filing: 09.01.2018
(51) Int. Cl.: C07C 323/22, C07C 59/84, A61K 31/192, A61K 31/10, A61P 1/16, A61P 3/06

(54) **CRYSTAL FORM OF GFT-505 AND PREPARATION METHOD AND USE THEREOF**

(30) Priority: 22.01.2017 CN 201710053395; 08.02.2017 CN 201710068280
(71) Applicant: Crystal Pharmaceutical (Suzhou) Co., Ltd., Suzhou, Jiangsu 215123 (CN)
(72) Inventor: CHEN, Minhua, Jiangsu 215123 (CN); ZHANG, Yanfeng, Jiangsu 215123 (CN); WANG, Jinqiu, Jiangsu 215123 (CN); LIU, Kai, Jiangsu 215123 (CN); ZHANG, Xiaoyu, Jiangsu 215123 (CN)
(74) Representative: Grünecker Patent- und Rechtsanwälte PartG mbB
(86) International application number: PCT/CN2018/071917
(87) International publication number: WO 2018/133705

(57) **Abstract**

The present disclosure relates to novel crystalline forms of GFT-505, processes for preparation and uses thereof. The crystalline form CS1, crystalline form CS2, crystalline form CS5 and crystalline form CS6 of GFT-505 provided by the present disclosure have high purity, good stability, low hygroscopicity, good solubility and good mechanical stability. The present disclosure provides a new and better choice for the preparation of drug products containing GFT-505 and is of great significance for drug development.

## Description

### TECHNICAL FIELD

The present disclosure relates to the field of pharmaceutical polymorphism. In particular, relates to novel crystalline forms of GFT-505, processes for preparation and use thereof.

### BACKGROUND

Non-alcoholic steatohepatitis (NASH) is a serious liver disease that pre-exists before hepatocellular carcinoma and is currently incurable. Elafibranor, also known as GFT-505, developed by Genfit and clinically used to treat NASH shows good safety and efficacy. GFT-505 is an agonist of activate peroxisome proliferator-activated receptor-α (PPARA) and receptor-δ (PPARD). Investigations have shown that GFT-505 can improve insulin sensitivity, glucose balance, lipid metabolism, and reduce inflammatory responses. GFT-505 is expected to be a new therapy for NASH. The chemical name of GFT-505 is 1-[4-methylthiophenyl]-3-[3, 5-dimethyl-4-carboxy-dimethylmethyloxyphenyl] prop-2-en-1-one and the structure is shown as compound (I):

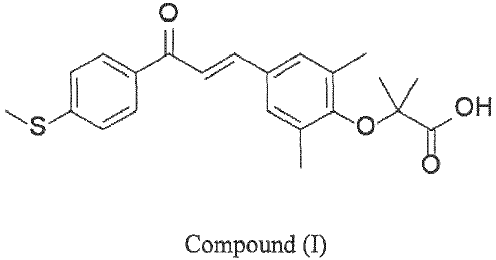

Different crystalline forms of solid chemical drugs can lead to differences in their solubility, stability, flowability and compressibility, thereby affecting the safety and efficacy of pharmaceutical products containing the compounds (see K. Knapman, *Modern Drug Discovery,* 3, 53-54,57,**2000**.), which results in differences in clinical efficacy. The discovery of new crystalline forms (including anhydrates, hydrates, solvates, etc.) of the active pharmaceutical ingredients may provide drug substance with processing advantages and better physical and chemical properties such as better bioavailability, better storage stability, easiness to process, and easiness to purify. Some novel crystalline forms may serve as intermediate crystal forms to facilitate solid state transformation to desired forms. Novel polymorphs of raw materials can enhance the performance of the drug and provide more solid states in the formulation.

CN100548960C disclosed the chemical structure and preparation method of GFT-505. The inventors of the present disclosure repeated the preparation method disclosed in the prior art and obtained a lightly yellow sticky oily substance which is difficult to be transferred and precisely quantified. Furthermore, the oily substance has low purity, poor stability and is difficult to be made in to drug products and not suitable for medical use. There is no report of GFT-505 crystalline forms so far. Therefore, it is in great need to investigate GFT-505 and find novel crystalline form which is superior in various aspects such as good stability, low hygroscopicity, high purity and so on to meet the need of industrial production and pharmaceutical preparations.

The inventors have accidentally discovered the crystal form CS1, crystal form CS2, crystal form CS5 and crystal form CS6 of the present disclosure by a large number of experiments. The crystal form CS1, crystal form CS2, crystal form CS5 and crystal form CS6 of GFT-505 provided by the present disclosure have high purity, good stability, low hygroscopicity, good solubility and high mechanical stability. The present disclosure provides a new and better choice for the preparation of drug products comprising GFT-505 and is of great significance for drug development.

### SUMMARY

The main objective of the present disclosure is to provide novel crystalline forms of GFT-505, processes for preparation and use thereof.

According to the objective of the present disclosure, crystalline form CS1 of Compound (I) is provided (hereinafter referred to as Form CS1), said Form CS1 is an anhydrate.

According to one aspect of the present disclosure, the X-ray powder diffraction pattern of Form CS1 shows characteristic peaks at 2theta values of 10.5°±0.2°, 14.8°±0.2° and 16.9°±0.2° using CuKα radiation.

Furthermore, the X-ray powder diffraction pattern of Form CS1 shows one or two or three characteristic peaks at 2theta values of 18.7°±0.2°, 20.4°±0.2°, 26.6°±0.2°. Preferably, the X-ray powder diffraction pattern of Form CS1 shows three characteristic peaks at 2theta values of 18.7°±0.2°, 20.4°±0.2°, 26.6°±0.2°.

Furthermore, the X-ray powder diffraction pattern of Form CS1 shows one or two or three characteristic peaks at 2theta values of 11.4°±0.2°, 23.5°±0.2°, 25.1°±0.2°. Preferably, the X-ray powder diffraction pattern of Form CS1 shows three characteristic peaks at 2theta values of 11.4°±0.2°, 23.5°±0.2°, 25.1°±0.2°.

In a preferred embodiment, the X-ray powder diffraction pattern of Form CS1 shows characteristic peaks at 2theta values of 10.5°±0.2°, 14.8°±0.2°, 16.9°±0.2°, 18.7°±0.2°, 20.4°±0.2°, 26.6°±0.2°, 11.4°±0.2°, 23.5°±0.2° and 25.1°±0.2°.

Furthermore, the X-ray powder diffraction pattern of Form CS1 shows one or two characteristic peaks at 2theta values of 8.0°±0.2°, 12.3°±0.2°. Preferably, the X-ray powder diffraction pattern of Form CS1 shows two characteristic peaks at 2theta values of 8.0°±0.2°, 12.3°±0.2°.

In a preferred embodiment, the X-ray powder diffraction pattern of Form CS1 shows characteristic peaks at 2theta values of 10.5°±0.2°, 14.8°±0.2°, 16.9°±0.2°, 18.7°±0.2°, 20.4°±0.2°, 26.6°±0.2°, 11.4°±0.2°, 23.5°±0.2°, 25.1°±0.2°, 8.0°±0.2°, 12.3°±0.2°.

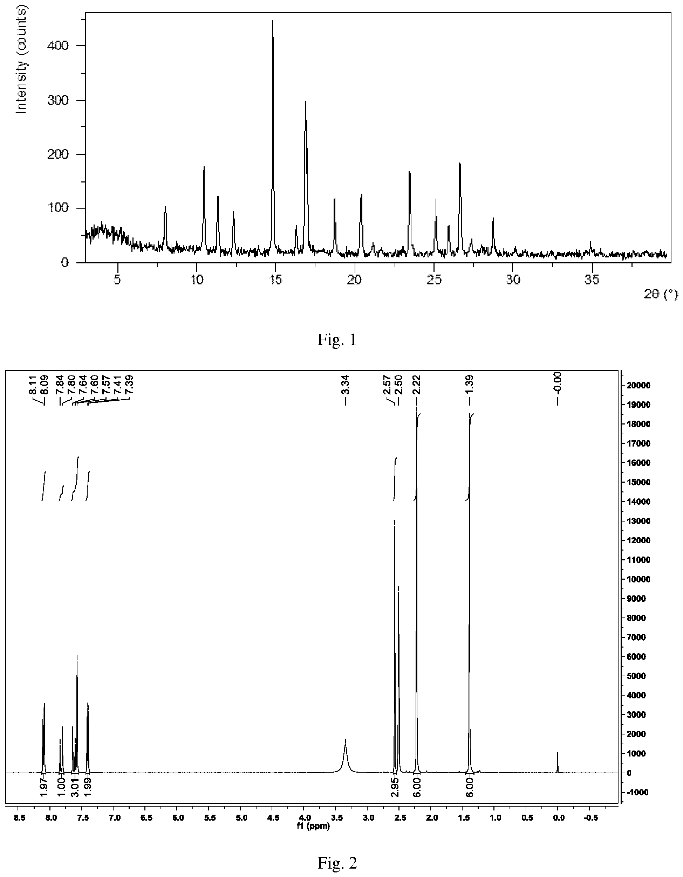

Without any limitation being implied, in a specific example of the present disclosure, the X-ray powder diffraction pattern of Form CS1 is substantially as depicted in FIG 1.

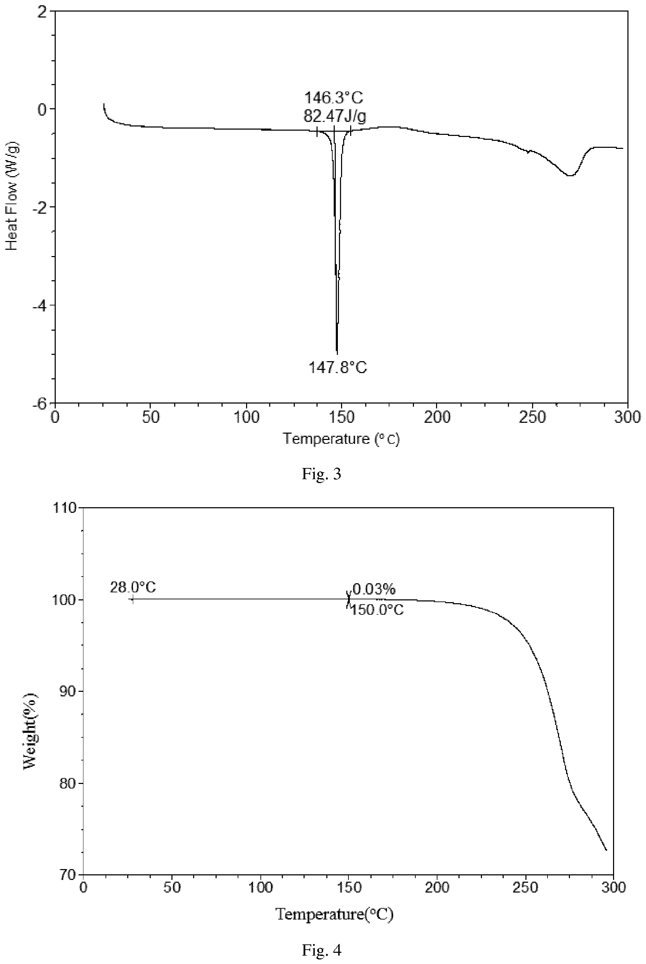

Without any limitation being implied, in a specific embodiment of the present disclosure, when differential scanning calorimetry analysis (DSC) is conducted for the Form CS1 of the present disclosure, the first endothermic peak corresponding to the melting of Form CS1 appears at around 146 °C. The DSC curve is depicted in FIG. 3.

Without any limitation being implied, in a specific embodiment of the present disclosure, when thermal gravimetric analysis (TGA) is conducted for the Form CS1 of the present disclosure, there is nearly no weight loss when heated to around 150 °C. The TGA curve is depicted in FIG. 4.

According to the objective of the present disclosure, a process for preparing Form CS1 is also provided. The process comprises the following method (1) or method (2):
(1) Dissolving GFT-505 in a solvent of ketones, adding anti-solvent, stirring, isolating and drying to obtain the solid. Wherein:
   Said ketone is a single solvent selected from C₃-C₅ ketones or a solvent mixture of C₃-C₅ ketones;
   Furthermore, said ketone includes acetone, methyl ethyl ketone or solvent mixture of acetone and methyl ethyl ketone. Preferably, said ketone is acetone.

   Said anti-solvent is a single solvent selected from C₅-C₉ alkanes or a solvent mixture of C₅∼C₉ alkanes;
   Furthermore, said alkane includes n-hexane, n-heptane, n-octane or a solvent mixture of n-hexane, n-heptane, and n-octane. Preferably, said alkane is n-heptane.
   Said volume ratio of ketone and alkane is 1:20-20:1. Preferably, said volume ratio is 1:20.
(2) Dissolving GFT-505 in a solvent mixture comprising aromatic hydrocarbons and ketones at high temperature, precipitating the crystal at low temperature, isolating and drying to obtain the solid. Wherein:
   Said aromatic hydrocarbon is a single solvent selected from C₇-C₉ aromatic hydrocarbons or a solvent mixture of C₇-C₉ aromatic hydrocarbons;
   Furthermore, said aromatic hydrocarbon includes toluene, ethyl benzene or a solvent mixture of toluene and ethyl benzene. Preferably, said aromatic hydrocarbon is toluene.

Said ketone is a single solvent selected from C₃-C₇ ketones or a solvent mixture of C₃-C₇ ketones;
Furthermore, said ketone includes acetone, methyl ethyl ketone, methyl isobutyl ketone or a solvent mixture of acetone, methyl ethyl ketone, and methyl isobutyl ketone. Preferably, said ketone is methyl isobutyl ketone.

Said volume ratio of aromatic hydrocarbon and ketone is 1:20-20:1, and preferably, said volume ratio is 13:1.

Said high temperature is 40-70 °C, said low temperature is 0-10 °C. Preferably, said high temperature is 50 °C, said low temperature is 4 °C.

According to the objective of the present disclosure, crystalline form CS2 of Compound (I) is provided (hereinafter referred to as Form CS2), said Form CS2 is a hydrate.

According to one aspect of the present disclosure, the X-ray powder diffraction pattern of Form CS2 shows characteristic peaks at 2theta values of 15.2°±0.2°, 15.9°±0.2° and 25.8°±0.2° using CuKα radiation.

Furthermore, the X-ray powder diffraction pattern of Form CS2 shows one or two or three characteristic peaks at 2theta values of 11.7°±0.2°, 12.2°±0.2°, 19.4°±0.2°. Preferably, the X-ray powder diffraction pattern of Form CS2 shows three characteristic peaks at 2theta values of 11.7°±0.2°, 12.2°±0.2°, 19.4°±0.2°.

Furthermore, the X-ray powder diffraction pattern of Form CS2 shows one or two or three characteristic peaks at 2theta values of 20.0°±0.2°, 26.8°±0.2°, 27.5°±0.2°. Preferably, the X-ray powder diffraction pattern of Form CS2 shows three characteristic peaks at 2theta values of 20.0°±0.2°, 26.8°±0.2°, 27.5°±0.2°.

In a preferred embodiment, the X-ray powder diffraction pattern of Form CS2 shows characteristic peaks at 2theta values of 15.2°±0.2°, 15.9°±0.2°, 25.8°±0.2°, 11.7°±0.2°, 12.2°±0.2°, 19.4°±0.2°, 20.0°±0.2°, 26.8°±0.2°, 27.5°±0.2°.

In a preferred embodiment, the X-ray powder diffraction pattern of Form CS2 shows characteristic peaks at 2theta values of 15.2°±0.2°, 15.9°±0.2°, 25.8°±0.2°, 11.7°±0.2°, 12.2°±0.2°, 19.4°±0.2°, 20.0°±0.2°, 26.8°±0.2°, 27.5°±0.2°, 14.7°±0.2°.

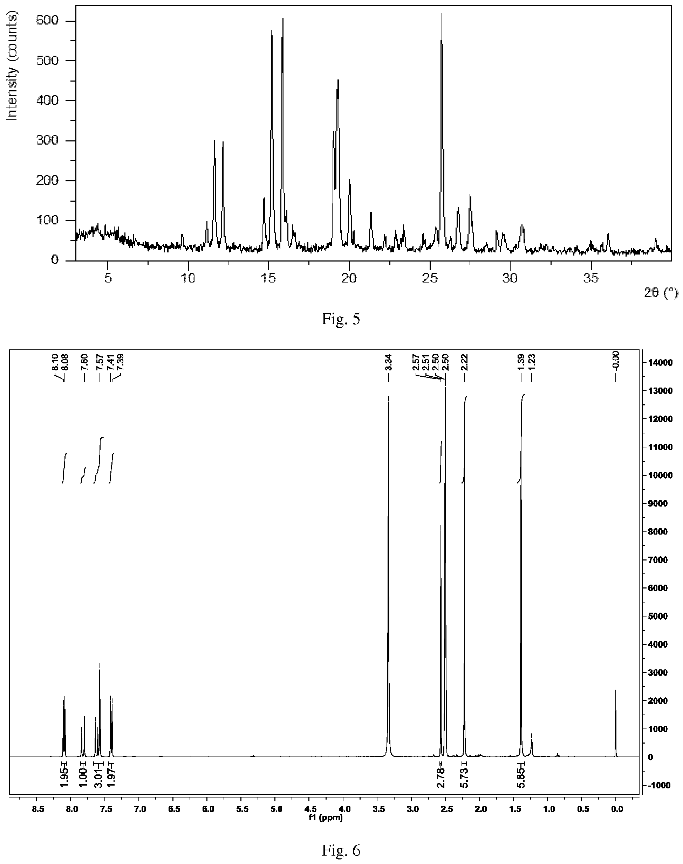

Without any limitation being implied, in a specific embodiment of the present disclosure, the X-ray powder diffraction pattern of Form CS2 is substantially as depicted in FIG 5.

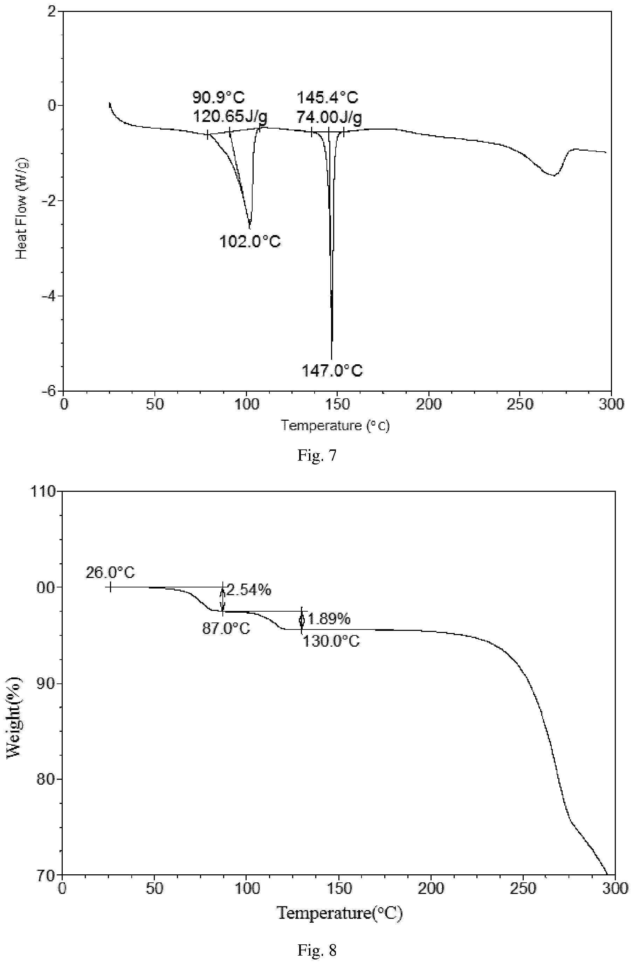

Without any limitation being implied, in a specific embodiment of the present disclosure, the DSC curve of Form CS2 is substantially as depicted in FIG. 7, which shows two endothermic peaks. The first endothermic peak is at around 91 °C (onset temperature), and the second endothermic peak is at around 145 °C (onset temperature).

Without any limitation being implied, in a specific embodiment of the present disclosure, the TGA curve of Form CS2 is substantially as depicted in FIG 8, which shows about 2.5 % weight loss when heated to 87 °C, and about 1.9 % more weight loss when further heated to 130 °C.

According to the objective of the present disclosure, a process for preparing Form CS2 is also provided. The process comprises the following method (1) or method (2) or method (3):
(1) Suspending GFT-505 in water or a solvent mixture comprising alcohols and water, stirring, isolating and drying to obtain the solid. Wherein:
   Said alcohol is a single solvent selected from C₁-C₅ alcohols or a solvent mixture of C₁-C₅ alcohols;
   Furthermore, said alcohol includes methanol, ethanol, isopropanol or a solvent mixture of methanol, ethanol, and isopropanol. Preferably, said alcohol is ethanol.

   Said volume ratio of alcohol and water is 1:5-5:1. Preferably, said volume ratio is 4:5.
(2) Dissolving GFT-505 in a solvent selected from alcohols, adding water as an anti-solvent, stirring, isolating and drying to obtain the solid.
   Wherein:
   Said alcohol is a single solvent selected from C₁-C₅ alcohols or a solvent mixture of C₁-C₅ alcohols;
   Furthermore, said alcohol includes a single solvent or a solvent mixture of methanol, ethanol, and isopropanol. Preferably, said alcohol is methanol.

   Said volume ratio of alcohol and water is 1:10-10:1. Preferably, said volume ratio is 1:7.
(3) Dissolving GFT-505 in a solvent mixture comprising alcohols and alkanes, adding polymer and evaporating at 10-50 °C to obtain the solid.

Wherein:
Said alcohol is a single solvent selected from C₁-C₅ alcohols or a solvent mixture of C₁-C₅ alcohols;
Furthermore, said alcohol includes methanol, ethanol, isopropanol or a solvent mixture ofmethanol, ethanol, and isopropanol, and preferably, said alcohol is ethanol.

Said alkane is a single solvent selected from C₆-C₉ alkanes or a solvent mixture of C₆-C₉ alkanes;
Furthermore, said alkane includes hexane, n-heptane, n-octane or a solvent mixture of hexane, n-heptane, and n-octane, and preferably, said alkane is n-heptane.

Furthermore, the volume ratio of said alcohol and alkane is 1:15-15:1. Preferably, the volume ratio is 15:4.

Furthermore, said polymer is a mixture of equal amount of polycaprolactone, polyoxyethylene, polymethyl methacrylate, hydroxyethyl cellulose, and sodium alginate.

Furthermore, said evaporation temperature is room temperature.

According to the objective of the present disclosure, crystalline form CS5 of Compound (I) is provided (hereinafter referred to as Form CS5), said Form CS5 is an anhydrate.

According to one aspect of the present disclosure, the X-ray powder diffraction pattern of Form CS5 shows characteristic peaks at 2theta values of 7.4°±0.2°, 14.6°±0.2° and 18.7°±0.2° using CuKα radiation.

Furthermore, the X-ray powder diffraction pattern of Form CS5 shows one or two or three characteristic peaks at 2theta values of 25.3°±0.2°, 15.4°±0.2°, 25.9°±0.2°. Preferably, the X-ray powder diffraction pattern of Form CS5 shows three characteristic peaks at 2theta values of 25.3°±0.2°, 15.4°±0.2°, 25.9°±0.2°.

Furthermore, the X-ray powder diffraction pattern of Form CS5 shows one or two or three characteristic peaks at 2theta values of 19.5°±0.2°, 27.5°±0.2°, 28.9°±0.2°. Preferably, the X-ray powder diffraction pattern of Form CS5 shows three characteristic peaks at 2theta values of 19.5°±0.2°, 27.5°±0.2°, 28.9°±0.2°.

In a preferred embodiment, the X-ray powder diffraction pattern of Form CS5 shows characteristic peaks at 2theta values of 7.4°±0.2°, 14.6°±0.2°, 18.7°±0.2°, 25.3°±0.2°, 15.4°±0.2°, 25.9°±0.2°, 19.5°±0.2°, 27.5°±0.2°, 28.9°±0.2°.

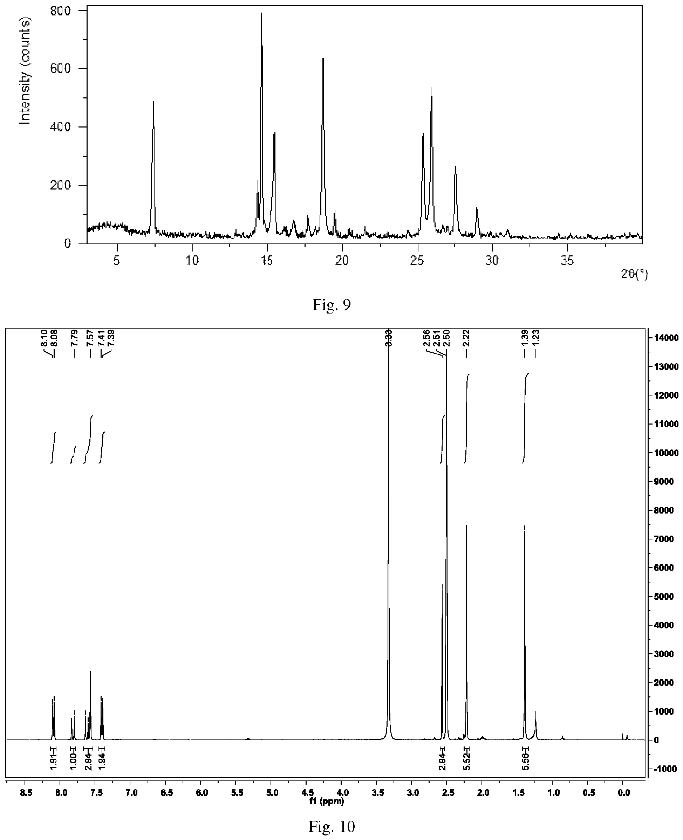

Without any limitation being implied, in a specific embodiment of the present disclosure, the X-ray powder diffraction pattern of Form CS5 is substantially as depicted in FIG 9.

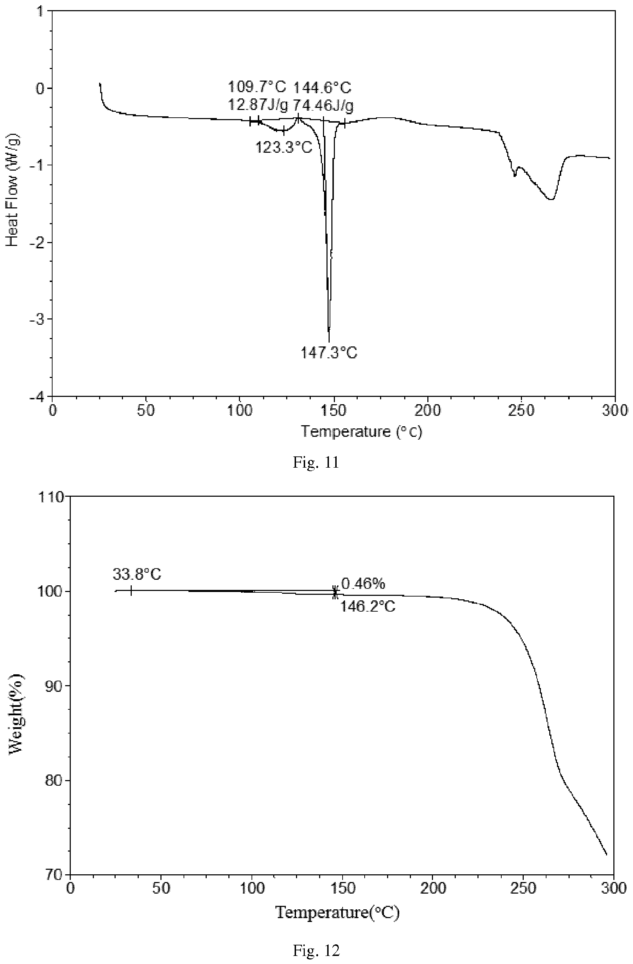

Without any limitation being implied, in a specific embodiment of the present disclosure, the DSC curve of Form CS5 is substantially as depicted in FIG. 11, which shows two endothermic peaks. The first endothermic peak is at around 110 °C (onset temperature), and the second endothermic peak is at around 145 °C (onset temperature).

Without any limitation being implied, in a specific embodiment of the present disclosure, the TGA curve of Form CS2 is substantially as depicted in FIG 12, which shows about 0.46 % weight loss when heated to 146 °C.

According to the objective of the present disclosure, a process for preparing Form CS5 is also provided. The process comprises:
Dissolving GFT-505 in a solvent mixture comprising ketones and aromatic hydrocarbons or a solvent mixture comprising esters and aromatic hydrocarbons, and evaporating at 10-50 °C to obtain the solid.

Wherein:
Said ketone is a single solvent selected from C₃-C₅ ketones or a solvent mixture of C₃-C₅ ketones;
Furthermore, said ketone includes acetone, methyl ethyl ketone or a solvent mixture of acetone and methyl ethyl ketone. Preferably, said ketone is acetone.

Said aromatic hydrocarbon is a single solvent selected from C₇-C₉ aromatic hydrocarbons or a solvent mixture of C₇-C₉ aromatic hydrocarbons;
Furthermore, said aromatic hydrocarbon includes toluene, ethyl benzene or a solvent mixture of toluene and ethyl benzene. Preferably, said aromatic hydrocarbon is toluene.

Said ester is a single solvent selected from C₃-C₆ esters or a solvent mixture of C₃-C₆ esters;
Furthermore, said ester includes ethyl acetate, isopropyl acetate or a solvent mixture of ethyl acetate and isopropyl acetate. Preferably, said ester is ethyl acetate.

Furthermore, said volume ratio of ketones and aromatic hydrocarbons or esters and aromatic hydrocarbons is 1:3-3:1. Preferably, said volume ratio is 1:1.

Furthermore, said evaporating temperature is room temperature.

According to the objective of the present disclosure, crystalline form CS6 of Compound (I) is provided (hereinafter referred to as Form CS6), said Form CS6 is an acetic acid solvate.

According to one aspect of the present disclosure, the X-ray powder diffraction pattern of Form CS6 shows characteristic peaks at 2theta values of 12.5°±0.2°, 19.4°±0.2° and 23.6°±0.2° using CuKα radiation.

Furthermore, the X-ray powder diffraction pattern of Form CS6 shows one or two or three characteristic peaks at 2theta values of 15.2°±0.2°, 20.7°±0.2°, 26.4°±0.2°. Preferably, the X-ray powder diffraction pattern of Form CS6 shows three characteristic peaks at 2theta values of 15.2°±0.2°, 20.7°±0.2°, 26.4°±0.2°.

Furthermore, the X-ray powder diffraction pattern of Form CS6 shows one or two or three characteristic peaks at 2theta values of 6.6°±0.2°, 10.3°±0.2°, 18.2°±0.2°. Preferably, the X-ray powder diffraction pattern of Form CS6 shows three characteristic peaks at 2theta values of 6.6°±0.2°, 10.3°±0.2°, 18.2°±0.2°.

In a preferred embodiment, the X-ray powder diffraction pattern of Form CS6 shows characteristic peaks at 2theta values of 12.5°±0.2°, 19.4°±0.2°, 23.6°±0.2°, 15.2°±0.2°, 20.7°±0.2°, 26.4°±0.2°, 6.6°±0.2°, 10.3°±0.2°, 18.2°±0.2°.

Furthermore, the X-ray powder diffraction pattern of Form CS6 shows one or more characteristic peaks at 2theta values of 11.1°±0.2°, 13.2°±0.2°, 16.2°±0.2°, 17.0°±0.2°, 25.1°±0.2°. Preferably, the X-ray powder diffraction pattern of Form CS6 shows characteristic peaks at 2theta values of 11.1°±0.2°, 13.2°±0.2°, 16.2°±0.2°, 17.0°±0.2°, 25.1°±0.2°.

In a preferred embodiment, the X-ray powder diffraction pattern of Form CS6 shows characteristic peaks at 2theta values of 12.5°±0.2°, 19.4°±0.2°, 23.6°±0.2°, 15.2°±0.2°, 20.7°±0.2°, 26.4°±0.2°, 6.6°±0.2°, 10.3°±0.2°, 18.2°±0.2°, 11.1°±0.2°, 13.2°±0.2°, 16.2°±0.2°, 17.0°±0.2°, 25.1°±0.2°.

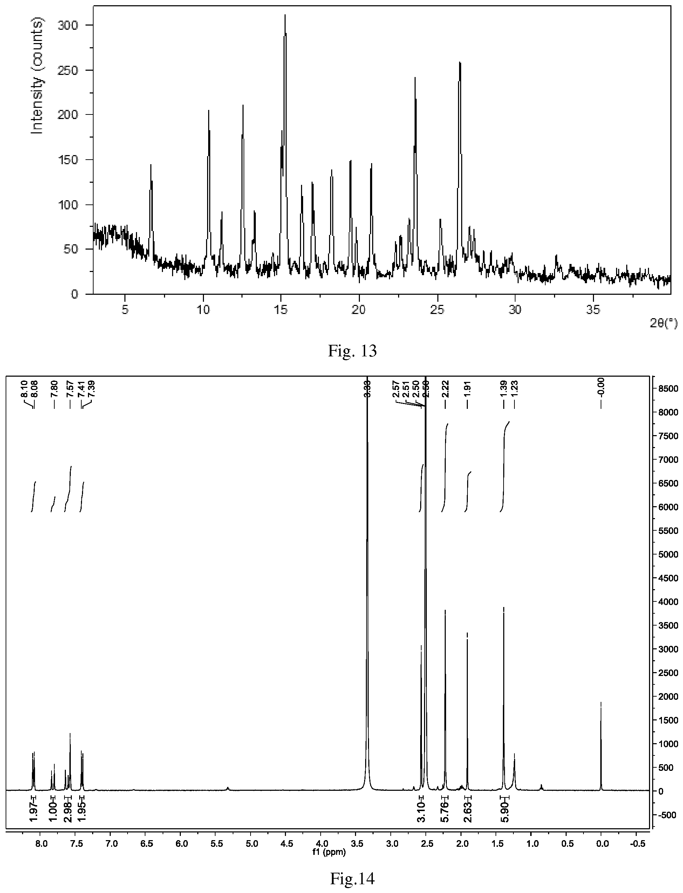

Without any limitation being implied, in a specific embodiment of the present disclosure, the X-ray powder diffraction pattern of Form CS6 is substantially as depicted in FIG 13.

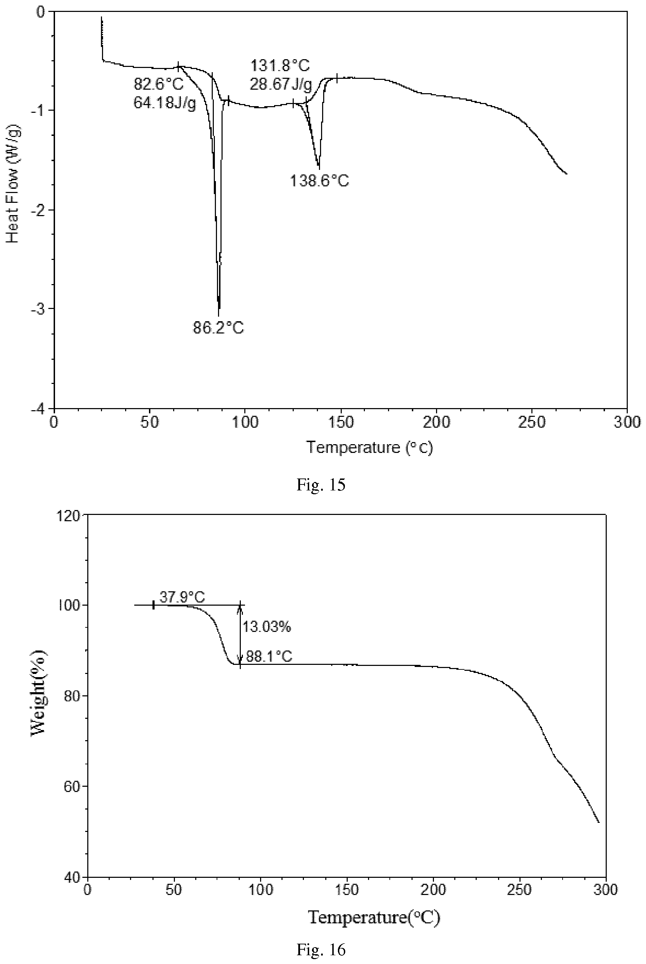

Without any limitation being implied, in a specific embodiment of the present disclosure, the DSC curve of Form CS6 is substantially as depicted in FIG. 15, which shows two endothermic peaks. The first endothermic peak is at around 83 °C (onset temperature), and the second endothermic peak is at around 132 °C (onset temperature).

Without any limitation being implied, in a specific embodiment of the present disclosure, the TGA curve of Form CS6 is substantially as depicted in FIG 16, which shows about 13.0 % weight loss when heated to 88 °C. There is 1.0 mole of acetic acid in Form CS6 calculated by TGA.

According to the objective of the present disclosure, a process for preparing Form CS6 is also provided. The process comprises:
Placing GFT-505 in a closed container which contains solvent atmosphere of acetic acid to obtain the solid by solid vapor diffusion.

According to the present disclosure, in the processes for preparing Form CS1, Form CS2, Form CS5 and Form CS6, said GFT-505 refers to solid form, semi-solid form, wax or oily substance of compound (I).

Said "temperature" is not a fixed value, but a temperature range from 10°C to 30°C.

In the present disclosure, "crystal" or "crystalline form" refers to the crystal or the crystal form being identified by the X-ray diffraction pattern shown herein. Those skilled in the art are able to understand that physicochemical properties discussed herein can be characterized, wherein the experimental errors depend on the instrument conditions, the sampling processes and the purity of samples. In particular, those skilled in the art generally know that the X-ray diffraction pattern typically varies with the experimental conditions. It is necessary to point out that, the relative intensity of the diffraction peaks in the X-ray diffraction pattern may also vary with the experimental conditions; therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and not intended to be used for absolute comparison. In addition, the experimental error of the diffraction peak angle is usually 5% or less, and the error of these angles should also be taken into account, and an error of ±0.2° is usually allowed. In addition, due to experimental factors such as sample thickness, the overall offset of the diffraction peak is caused, and a certain offset is usually allowed. Thus, it will be understood by those skilled in the art that a crystalline form of the present disclosure is not necessarily to have the exactly same X-ray diffraction pattern of the example shown herein. As used herein, "the same XRPD pattern" does not mean absolutely the same, the same peak positions may differ by ±0.2° and the peak intensity allows for some variability. Any crystalline forms whose X-ray diffraction patterns have the same or similar characteristic peaks should be within the scope of the present disclosure. Those skilled in the art can compare the patterns shown in the present disclosure with that of an unknown crystalline form in order to identify whether these two groups of patterns reflect the same or different crystalline forms.

"Crystalline form" and "polymorphic form" as well as other related terms in the present disclosure refer to a specific crystal form of solid compounds. Physiochemical properties including stability during storage, compressibility, density, dissolution rate, etc. may be different in different polymorphs. In extreme cases, the difference in solubility or dissolution rate may result in drugs with low efficacy and even toxicity.

In some embodiments, Form CS1, Form CS2, Form CS5 and Form CS6 of the present disclosure are pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and further more specifically less than 1% (w/w).

It should be noted that the number and the number range should not be understood as the number or number range themselves only. It should be understood by those skilled in the art that the specific number can be shifted at specific technical environment without departing from the spirit and principle of the present disclosure. In the present disclosure, the number of shift ranges expected by one of skilled in the art is represented by the term "about".

In the present disclosure, said "anti-solvent" is the poor solvent of compound (I). Said "solid vapor diffusion" is storing the starting material in a hermetical condition which contains a specific solvent atmosphere. In solid vapor diffusion method, starting material is not contact with solvent directly. The novel polymorph is obtained by contacting of solvent vapor and starting material.

Form CS1, Form CS2, Form CS5 and Form CS6 of the present disclosure have the following advantages:
(1) The crystal forms of the present disclosure have a significantly improved purity compared to oily substance in the prior art. The purity of the prior art oily substance is only 83.87%, while the crystal forms of the present disclosure have higher purity. In a specific embodiment, the crystalline form of the present disclosure has a purity of higher than 98%. In another specific embodiment, the crystalline form of the present disclosure has a purity of higher than 99%. The crystal forms of the present disclosure have good purification effect. A purer drug substance can be obtained by a crystallization process, and the problem of solvent residue is less likely to occur. In this way, the sample residual solvent is easy to meet the standard and the quality requirements, which is suitable for medicinal use;
(2) The crystal forms provided by the present disclosure have lower hygroscopicity, which can overcome the disadvantages caused by high hygroscopicity, such as the uncertainty of the content of crystalline drug substance caused by the weight change due to water absorption and is beneficial to long-term storage of the drug products, reducing material storage and quality control costs. The weight gain of Form CS1, CS2 and CS5 of the present disclosure under the condition of 80% relative humidity (RH) is 0.042%, 0.101% and 0.325%, respectively. Form CS1, CS2 and CS5 have low hygroscopicity. Due to the low hygroscopicity, instability during drug preparation and/or storage and the un-processability of formulation caused by external factors such as environmental moisture can be avoided. Low hygroscopicity is advantageous for accurate quantification and later transportation and storage of the drug;
(3) The crystal forms of the present disclosure have good stability, thereby ensuring that the quality standard of the sample is consistent and controllable, and meets the stringent requirements for the crystal form in the pharmaceutical application and the preparation process. The crystalline structures of Form CS1, CS2 and CS5 of the present disclosure don't change for at least 1 months when stored under the condition of 25 °C/60% RH and/or 60 °C/75% RH, preferably for at least 6 months, more preferably for at least 1 year. Therefore, the crystal forms CS1, CS2 and CS5 of the present invention have good stability, which is favorable for storage of the sample and stability of the formulation;
(4) The crystalline forms provided by the invention has good solubility, can reduce the dosage of the medicine, thereby reducing the side effects of the medicine and improving the safety of the medicine, can achieve the required therapeutic blood concentration without a high dose after oral administration, and is beneficial to absorption in the human body to achieve the desired bioavailability and efficacy of the drug, and meet medicinal requirements
(5) The crystal form provided by the present disclosure has good mechanical stability. Good mechanical stability of the drug substance can reduce the risk of crystallinity decrease and crystal transformation during the drug production process. Form CS1, Form CS2, Form CS5 and Form CS6 of the present disclosure have good mechanical stability. Grinding and pulverization are often required in the drug manufacturing process. Good mechanical stability of the drug substance can reduce the risk of crystallinity decrease and crystal transformation during the drug production process.

According to the objective of the present disclosure, a pharmaceutical composition is provided, said pharmaceutical composition comprises a therapeutically effective amount of Form CS1, Form CS2, Form CS5 and Form CS6 and at least one pharmaceutically acceptable excipients.

Furthermore, Form CS1, Form CS2, Form CS5 and Form CS6 of GFT-505 provided by the present disclosure can be used for preparing drugs treating NASH and/or type 2 diabetes and/or dyslipidemia and/or atherosclerosis.

### BRIEF DESCRIPTION OF THE DRAWINGS

FIG. 1 shows an XRPD pattern of Form CS1 according to example 1.
FIG. 2 shows a ¹H NMR spectrum of Form CS1 according to example 1.
FIG. 3 shows a DSC curve of Form CS1 according to example 1.
FIG. 4 shows a TGA curve of Form CS1 according to example 1.
FIG. 5 shows an XRPD pattern of Form CS2 according to example 4.
FIG. 6 shows a ¹H NMR spectrum of Form CS2 according to example 4.
FIG. 7 shows a DSC curve of Form CS2 according to example 4.
FIG. 8 shows a TGA curve of Form CS2 according to example 4.
FIG. 9 shows an XRPD pattern of Form CS5 according to example 7.
FIG. 10 shows a ¹H NMR spectrum of Form CS5 according to example 7.
FIG. 11 shows a DSC curve of Form CS5 according to example 7.
FIG. 12 shows a TGA curve of Form CS5 according to example 7.
FIG. 13 shows an XRPD pattern of Form CS6 according to example 9.
FIG. 14 shows a ¹H NMR spectrum of Form CS6 according to example 9.
FIG 15 shows a DSC curve of Form CS6 according to example 9.
FIG. 16 shows a TGA curve of Form CS6 according to example 9.
FIG. 17 shows a DVS plot of Form CS1.
FIG. 18 shows an XRPD pattern overlay of Form CS1 before and after DVS test (top: XRPD pattern before DVS; bottom: XRPD pattern after DVS)
FIG. 19 shows a DVS plot of Form CS2.
FIG. 20 shows an XRPD pattern overlay of Form CS2 before and after DVS test (top: XRPD pattern before DVS; bottom: XRPD pattern after DVS)
FIG. 21 shows a DVS plot of Form CS5.
FIG. 22 shows an XRPD pattern overlay of Form CS5 before and after DVS test (top: XRPD pattern before DVS; bottom: XRPD pattern after DVS).
FIG. 23 shows an XRPD pattern overlay of Form CS1 before and after stored at 25 °C/60% RH for 6 months (top: XRPD pattern before stored at 25 °C/60% RH, bottom: XRPD pattern after stored at 25 °C/60% RH)
FIG. 24 shows an XRPD pattern overlay of Form CS1 before and after stored at 40 °C/75% RH for 6 months (top: XRPD pattern before stored at 40 °C/75% RH, bottom: XRPD pattern after stored at 40 °C/75% RH)
FIG. 25 shows an XRPD pattern overlay of Form CS1 before and after stored at 60 °C/75% RH for 1 months (top: XRPD pattern before stored at 60 °C/75% RH, bottom: XRPD pattern after stored at 60 °C/75% RH)
FIG. 26 shows an XRPD pattern overlay of Form CS2 before and after stored at 25 °C/60% RH for 6 months (top: XRPD pattern before stored at 25 °C/60% RH, bottom: XRPD pattern after stored at 25 °C/60% RH)
FIG. 27 shows an XRPD pattern overlay of Form CS2 before and after stored at 40 °C/75% RH for 6 months (top: XRPD pattern before stored at 40 °C/75% RH, bottom: XRPD pattern after stored at 40 °C/75% RH)
FIG. 28 shows an XRPD pattern overlay of Form CS2 before and after stored at 60 °C/75% RH for 1 months (top: XRPD pattern before stored at 60 °C/75% RH, bottom: XRPD pattern after stored at 60 °C/75% RH)
FIG. 29 shows an XRPD pattern overlay of Form CS5 before and after stored at 25 °C/60% RH for 6 months (top: XRPD pattern before stored at 25 °C/60% RH, bottom: XRPD pattern after stored at 25 °C/60% RH)
FIG. 30 shows an XRPD pattern overlay of Form CS5 before and after stored at 40 °C/75% RH for 6 months (top: XRPD pattern before stored at 40 °C/75% RH, bottom: XRPD pattern after stored at 40 °C/75% RH)
FIG. 31 shows an XRPD pattern overlay of Form CS5 before and after stored at 60 °C/75% RH for 1 months (top: XRPD pattern before stored at 60 °C/75% RH, bottom: XRPD pattern after stored at 60 °C/75% RH).
FIG. 32 shows an XRPD pattern overlay of Form CS1 before and after grinding (top: XRPD pattern before grinding, bottom: XRPD pattern after grinding).
FIG. 33 shows an XRPD pattern overlay of Form CS2 before and after grinding (top: XRPD pattern before grinding, bottom: XRPD pattern after grinding).
FIG. 34 shows an XRPD pattern overlay of Form CS5 before and after grinding (top: XRPD pattern before grinding, bottom: XRPD pattern after grinding).
FIG. 35 shows an XRPD pattern overlay of Form CS6 before and after grinding (top: XRPD pattern before grinding, bottom: XRPD pattern after grinding).
FIG. 36 shows morphology comparison of Form CS1 and oily substance. (left: morphology of Form CS1 of the present disclosure, right: morphology of oily substance prepared from the prior art).

### DETAILED DESCRIPTION

The present disclosure is further illustrated by the following examples which describe the preparation and use of the crystalline forms of the present disclosure in detail. It is obvious to those skilled in the art that many changes in the materials and methods can be accomplished without departing from the scope of the present disclosure.

The abbreviations used in the present disclosure are explained as follows:
XRPD: X-ray Powder Diffraction
DSC: Differential Scanning Calorimetry
TGA: Thermal Gravimetric Analysis
DVS: Dynamic Vapor Sorption
HPLC: High Performance Liquid Chromatography
¹H NMR: Proton Nuclear Magnetic Resonance
PSD: Particle Size Distribution
Instruments and methods used for data collection

X-ray powder diffraction patterns in the present disclosure were acquired by a Bruker D2 PHASER X-ray powder diffractometer. The parameters of the X-ray powder diffraction method of the present disclosure are as follows:
X-ray Reflection: Cu, Kα
Kα1 (Å) : 1.540598 ; Kα2 (Å) : 1.544426
Kα2/Kα1 intensity ratio: 0.50
Voltage: 45 (kV)
Current: 40 (mA)
Scan range: from 3.0 degree to 40.0 degree

Differential scanning calorimetry (DSC) data in the present disclosure were acquired by a TA Q2000. The parameters of the DSC method of the present disclosure are as follows:
Heating rate: 10 °C/min unless otherwise specified.
Purge gas: nitrogen

Thermal gravimetric analysis (TGA) data in the present disclosure were acquired by a TA Q500. The parameters of the TGA method of the present disclosure were as follows:
Heating rate: 10 °C/ min
Purge gas: nitrogen
High Performance Liquid Chromatography (HPLC) data in the present disclosure were collected from an Agilent 1260 with Ultraviolet variable wavelength detector (VWD).

The HPLC method parameters for purity test in the present disclosure are as follows:
1. Column: Waters XBridge C18 150×4.6mm, 5 µm
2. Mobile Phase:
A: 0.1% Phosphate in H₂O
B: Acetonitrile

**Gradient:**

| Time (min) | %B |
|---|---|
| 0.0 | 25 |
| 25.0 | 80 |
| 30.0 | 80 |
| 30.1 | 25 |
| 35.0 | 25 |

3. Flow rate: 1.0 mL/min
4. Injection Volume: 5 µL
5. Detection wavelength: 350 nm
6. Column Temperature: 40 °C
7. Diluent: Acetonitrile

Dynamic Vapor Sorption (DVS) was measured via an SMS (Surface Measurement Systems Ltd.) Intrinsic DVS instrument. Typical Parameters for DVS test are as follows:
Temperature: 25 °C
Gas and flow rate: N₂, 200 mL/min
dm/dt: 0.002%/min
RH range: 0% RH to 95% RH

Proton nuclear magnetic resonance spectrum data (¹H NMR) were collected from a Bruker Avance II DMX 400M HZ NMR spectrometer. 1-5 mg of sample was weighed, and dissolved in 0.5 mL of deuterated dimethyl sulfoxide to obtain a solution with a concentration of 2-10 mg/mL.

The particle size distribution data in the present disclosure were acquired by an S3500 laser particle size analyzer of Microtrac. Microtrac S3500 is equipped with an SDC (Sample Delivery Controller). The test is carried out in wet mode, and the dispersion medium is Isopar G (0.2% lecithin).

Unless otherwise specified, the following examples were conducted at room temperature.

Raw materials of GFT-505 used in the following examples were prepared by known methods in the prior art, for example, the method disclosed in CN100548960C.

### Example 1

7.2 mg of GFT-505 was dissolved in 0.1 mL of acetone and the solution was filtered. 2.0 mL of n-heptane was added into the filtrate drop by drop as an anti-solvent, followed by stirring at room temperature. The solid was collected, centrifuged and vacuum dried at RT. The obtained solid was confirmed to be Form CS1 of the present disclosure, of which the XRPD pattern is depicted in FIG. 1 and the XRPD data are listed in Table 1.

The ¹H NMR data of Form CS1 obtained in example 1 are as follow: ¹HNMR (400 MHz, DMSO) 8 8.10 (d, J = 8.5 Hz, 2H), 7.82 (d, J = 15.5 Hz, 1H), 7.66-7.54 (m, 3H), 7.40 (d, J = 8.5 Hz, 2H), 2.57 (s, 3H), 2.22 (s, 6H), 1.39 (s, 6H). The ¹H NMR spectrum is substantially as depicted FIG. 2.

The DSC curve of Form CS1 obtained in example 1 is depicted in FIG. 3, and the TGA curve is displayed in FIG. 4.

**Table 1**

| 2θ (±0.2°) | d spacing | Intensity % |
|---|---|---|
| 8.02 | 11.03 | 17.70 |
| 10.45 | 8.47 | 35.74 |
| 11.35 | 7.80 | 23.35 |
| 12.34 | 7.17 | 17.06 |
| 14.82 | 5.98 | 100.00 |
| 16.31 | 5.44 | 10.64 |
| 16.89 | 5.25 | 64.41 |
| 17.02 | 5.21 | 42.36 |
| 18.73 | 4.74 | 23.77 |
| 20.40 | 4.35 | 24.78 |
| 23.46 | 3.79 | 35.86 |
| 25.12 | 3.54 | 20.07 |
| 25.92 | 3.44 | 11.30 |
| 26.63 | 3.35 | 39.14 |
| 27.35 | 3.26 | 5.27 |
| 28.76 | 3.10 | 14.05 |

### Example 2

20.5 mg of GFT-505 was dissolved in 0.7 mL of solvent mixture of toluene and methyl isobutyl ketone (the volume ratio of toluene and methyl isobutyl ketone is 13:1) at 50 °C. The solution was filtered and cooled to 4 °C to precipitate the crystal, and the crystalline solid was obtained by centrifuging and vacuum drying at room temperature. The crystalline solid was characterized to be Form CS1 of the present disclosure, of which the XRPD data are listed in Table 2.

**Table 2**

| 2θ (±0.2°) | d spacing | Intensity % |
|---|---|---|
| 3.38 | 26.14 | 0.40 |
| 8.04 | 11.00 | 26.15 |
| 10.45 | 8.46 | 50.11 |
| 11.35 | 7.79 | 37.94 |
| 12.36 | 7.16 | 18.40 |
| 14.84 | 5.97 | 100.00 |
| 16.30 | 5.44 | 15.31 |
| 16.90 | 5.25 | 99.98 |
| 18.75 | 4.73 | 42.21 |
| 20.42 | 4.35 | 29.56 |
| 23.45 | 3.79 | 43.38 |
| 25.11 | 3.55 | 16.15 |
| 25.92 | 3.44 | 16.19 |
| 26.68 | 3.34 | 48.48 |
| 28.76 | 3.10 | 14.39 |

### Example 3

1.0 g of GFT-505 was suspended in 4.5 mL of solvent mixture of ethanol and water (the volume ratio of ethanol and water was 4:5) and the suspension was stirred at room temperature. Form CS2 of the present disclosure was obtained by isolating and vacuum drying. The XRPD pattern is depicted in FIG. 5. The XRPD data are listed in Table 3.

The ¹H NMR data of Form CS2 obtained in this example are as follow: ¹HNMR (400 MHz, DMSO) δ 8.09 (d, J = 8.5 Hz, 2H), 7.82 (d, J = 15.5 Hz, 1H), 7.67-7.52 (m, 3H), 7.40 (d, J = 8.5 Hz, 2H), 2.57 (s, 3H), 2.22 (s, 6H), 1.39 (s, 6H). The ¹H NMR spectrum is displayed in FIG. 6.

The DSC curve of Form CS2 obtained in this example is depicted in FIG. 7, and the TGA curve is displayed in FIG. 8.

**Table 3**

| 2θ (±0.2°) | d spacing | Intensity % |
|---|---|---|
| 9.68 | 9.13 | 3.76 |
| 11.17 | 7.92 | 9.76 |
| 11.66 | 7.59 | 41.04 |
| 12.17 | 7.28 | 44.01 |
| 14.74 | 6.01 | 19.59 |
| 15.21 | 5.82 | 93.64 |
| 15.89 | 5.58 | 99.14 |
| 16.53 | 5.36 | 9.11 |
| 19.06 | 4.66 | 51.10 |
| 19.24 | 4.61 | 63.21 |
| 19.35 | 4.59 | 66.96 |
| 20.02 | 4.43 | 29.96 |
| 21.38 | 4.16 | 16.10 |
| 22.20 | 4.00 | 5.34 |
| 22.91 | 3.88 | 6.45 |
| 23.40 | 3.80 | 7.62 |
| 24.63 | 3.61 | 5.48 |
| 25.39 | 3.51 | 9.84 |
| 25.75 | 3.46 | 100.00 |
| 26.29 | 3.39 | 5.19 |
| 26.77 | 3.33 | 18.14 |
| 27.50 | 3.24 | 23.27 |
| 29.19 | 3.06 | 7.80 |
| 29.53 | 3.03 | 5.87 |
| 30.75 | 2.91 | 10.29 |
| 36.09 | 2.49 | 7.44 |

### Example 4

103.5 mg of GFT-505 was dissolved in 1.0 mL of methanol, and 7.0 mL of water was added at room temperature as an anti-solvent. The solid was centrifuged and dried after stirring for 24 hours, and was characterized to be Form CS2 of the present disclosure. The XRPD data are listed in Table 4.

**Table 4**

| 2θ (±0.2°) | d spacing | Intensity % |
|---|---|---|
| 9.65 | 9.16 | 7.78 |
| 11.17 | 7.92 | 10.05 |
| 11.63 | 7.61 | 48.14 |
| 12.16 | 7.28 | 28.74 |
| 14.72 | 6.02 | 16.51 |
| 15.21 | 5.83 | 67.12 |
| 15.90 | 5.57 | 98.50 |
| 16.51 | 5.37 | 10.32 |
| 19.03 | 4.66 | 42.92 |
| 19.33 | 4.59 | 100.00 |
| 20.00 | 4.44 | 38.97 |
| 21.37 | 4.16 | 10.01 |
| 22.19 | 4.01 | 4.30 |
| 23.40 | 3.80 | 8.33 |
| 25.75 | 3.46 | 77.65 |
| 26.75 | 3.33 | 16.29 |
| 27.50 | 3.24 | 18.33 |
| 29.16 | 3.06 | 6.87 |
| 30.72 | 2.91 | 7.29 |
| 32.17 | 2.78 | 2.39 |
| 35.96 | 2.50 | 1.75 |

### Example 5

7.5 mg of GFT-505 was dissolved in 0.6 mL of solvent mixture of ethanol and n-heptane (the volume ratio of ethanol and n-heptane was 15:4). The solution was filtrated. Polymer comprising equal amount of polycaprolactone, polyoxyethylene, polymethyl methacrylate, hydroxyethyl cellulose, and sodium alginate was added into the filtrate. The solution was sealed with perforated Parafilm and evaporated at room temperature for about 5 days to get solid. The solid was confirmed to be Form CS2, and XRPD data of which are listed in Table 5.

**Table 5**

| 2θ (±0.2°) | d spacing | Intensity % |
|---|---|---|
| 11.18 | 7.92 | 5.66 |
| 11.65 | 7.60 | 22.81 |
| 12.15 | 7.29 | 26.34 |
| 14.72 | 6.02 | 15.20 |
| 15.21 | 5.82 | 65.10 |
| 15.89 | 5.58 | 56.45 |
| 16.51 | 5.37 | 7.81 |
| 19.03 | 4.66 | 43.77 |
| 19.31 | 4.60 | 74.42 |
| 20.02 | 4.43 | 19.48 |
| 21.39 | 4.15 | 16.80 |
| 22.20 | 4.00 | 5.29 |
| 22.91 | 3.88 | 7.88 |
| 24.63 | 3.61 | 6.62 |
| 25.80 | 3.45 | 100.00 |
| 26.77 | 3.33 | 14.92 |
| 27.55 | 3.24 | 24.47 |
| 29.61 | 3.02 | 6.88 |
| 30.87 | 2.90 | 10.45 |
| 35.06 | 2.56 | 4.92 |
| 36.10 | 2.49 | 6.77 |
| 39.07 | 2.31 | 6.25 |

### Example 6

6.4 mg of GFT-505 was dissolved in 0.6 mL of solvent mixture comprising acetone and toluene (the volume ratio of acetone and toluene was 1:1). The solution was filtrated and sealed with perforated parafilm. The filtrate was evaporated at room temperature for about 3 days, and the solid was characterized to be Form CS5 of the present disclosure. The XRPD pattern is displayed in FIG. 9 and the XRPD data are listed in Table 6.

The ¹H NMR data of the Form CS5 in this example are as follow: ¹HNMR (400 MHz, DMSO) δ 8.09 (d, J = 8.5 Hz, 2H), 7.81 (d, J = 15.6 Hz, 1H), 7.66-7.53 (m, 3H), 7.40 (d, J = 8.5 Hz, 2H), 2.56 (s, 3H), 2.22 (s, 6H), 1.39 (s, 6H). The ¹H NMR spectrum is displayed in FIG. 10.

The DSC curve of Form CS5 in this example is depicted in FIG. 11, and the TGA curve is displayed in FIG. 12.

**Table 6**

| 2θ (±0.2°) | d spacing | Intensity % |
|---|---|---|
| 7.37 | 11.99 | 57.43 |
| 14.32 | 6.19 | 21.43 |
| 14.58 | 6.08 | 100.00 |
| 15.44 | 5.74 | 45.41 |
| 16.71 | 5.31 | 6.78 |
| 17.66 | 5.02 | 9.37 |
| 18.68 | 4.75 | 79.74 |
| 19.47 | 4.56 | 10.85 |
| 21.48 | 4.14 | 2.85 |
| 25.32 | 3.52 | 43.73 |
| 25.85 | 3.45 | 64.55 |
| 27.51 | 3.24 | 30.47 |
| 28.88 | 3.09 | 12.67 |

### Example 7

18.5 mg of GFT-505 was dissolved in 1.5 mL of solvent mixture comprising acetone and toluene (the volume ratio of acetone and toluene was 1:2), then the solution was filtrated and sealed with perforated Parafilm. The filtrate was evaporated at room temperature for about 10 days, and the solid was characterized to be Form CS5, XRPD data of which are listed in Table 7.

**Table 7**

| 2θ (±0.2°) | d spacing | Intensity % |
|---|---|---|
| 7.26 | 12.17 | 46.05 |
| 14.56 | 6.08 | 71.42 |
| 15.18 | 5.84 | 31.66 |
| 15.43 | 5.74 | 22.46 |
| 16.78 | 5.28 | 5.44 |
| 17.56 | 5.05 | 21.90 |
| 18.71 | 4.74 | 100.00 |
| 19.47 | 4.56 | 19.66 |
| 20.31 | 4.37 | 1.56 |
| 21.58 | 4.12 | 3.12 |
| 22.03 | 4.04 | 4.32 |
| 22.98 | 3.87 | 3.76 |
| 24.24 | 3.67 | 5.48 |
| 25.44 | 3.50 | 8.89 |
| 26.00 | 3.43 | 14.08 |
| 26.81 | 3.33 | 2.82 |
| 27.60 | 3.23 | 9.54 |
| 28.86 | 3.09 | 14.67 |

### Example 8

8.0 mg of GFT-505 was dissolved in 0.5 mL of solvent mixture comprising ethyl acetate and toluene (the volume ratio of ethyl acetate and toluene was 1:1), then the solution was filtered and sealed with perforated Parafilm. The filtrate was evaporated at room temperature for about 3 days, and the solid was characterized to be Form CS5.

### Example 9

10.9 mg of GFT-505 was weighed into a 3-mL glass vial. The 3-mL vial was placed into the 20-mL glass vial which contained 3 mL of acetic acid. The 20-mL vial was sealed and standing for one week, then the solid was collected.

The solid was characterized to be Form CS6, XRPD pattern of which is displayed in FIG. 13 and the XRPD data are listed in Table 8.

The ¹H NMR data of Form CS6 in this example are as follow: ¹HNMR (400 MHz, DMSO) δ 8.09 (d, *J* = 8.6 Hz, 2H), 7.81 (d, *J* = 15.5 Hz, ¹H), 7.57 (s, 3H), 7.40 (d, *J* = 8.6 Hz, 2H), 2.57 (s, 3H), 2.22 (s, 6H), 1.91 (s, 3H), 1.39 (s, 6H). The ¹H NMR spectrum is displayed in FIG. 14.

The DSC curve is depicted in FIG. 15, and the TGA curve is displayed in FIG. 16.

**Table 8**

| 2θ (±0.2°) | d spacing | Intensity % |
|---|---|---|
| 6.60 | 13.40 | 31.51 |
| 10.33 | 8.57 | 61.94 |
| 11.14 | 7.94 | 20.54 |
| 12.50 | 7.08 | 63.30 |
| 13.25 | 6.68 | 20.50 |
| 14.97 | 5.92 | 51.20 |
| 15.19 | 5.83 | 100.00 |
| 16.24 | 5.46 | 31.07 |
| 16.96 | 5.23 | 35.83 |
| 18.21 | 4.87 | 37.41 |
| 19.39 | 4.58 | 43.47 |
| 19.76 | 4.49 | 14.69 |
| 20.74 | 4.28 | 42.06 |
| 22.59 | 3.94 | 13.99 |
| 23.13 | 3.85 | 21.58 |
| 23.55 | 3.78 | 68.43 |
| 25.11 | 3.55 | 16.03 |
| 26.38 | 3.38 | 81.16 |
| 26.99 | 3.30 | 19.01 |
| 29.68 | 3.01 | 7.79 |
| 32.64 | 2.74 | 3.95 |

### Example 10: Purity comparison among oily substance of the prior art, Form CS1 and Form CS2 of the present disclosure

The purities of oily substance prepared according to the prior art, Form CS1 of the present disclosure and Form CS2 of the present disclosure were measured by HPLC, and the results are listed in Table 9.

**Table 9**

| Form | Oily substance | Form CS1 | Form CS2 |
|---|---|---|---|
| Purity | 83.87% | 99.25% | 99.56% |

The purity of drug substance is significant for ensuring the drug efficacy and safety and preventing adverse side effect. The impurity content in oily substance of the prior art is extremely high, which may lead to obviously low drug substance content and decrease of activity. High impurity may result in a significant increase in toxicity and side effects as well, thus the oily substance of the prior art cannot be used as active pharmaceutical ingredient in the preparation of drug products.

The purity of the crystalline forms of the present disclosure is relatively high, which is beneficial for industrial production. The crystalline forms of the present disclosure have better purification effect. Drug substance with higher purity can be obtained by crystallization process with less residual solvent problems. The residual solvent of the sample is easy to meet the standard and the quality requirements, which is suitable for medicinal use.

### Example 11: Hygroscopicity study of Form CS1, Form CS2 and Form CS5

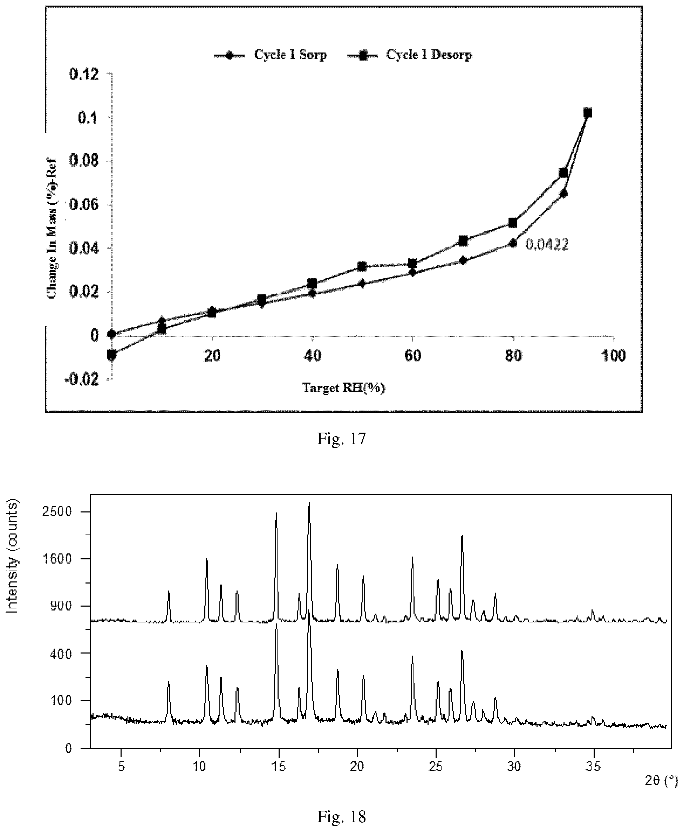

Dynamic vapor sorption (DVS) was applied to test hygroscopicity of Form CS1, Form CS2 and Form CS5 of the present disclosure with about 10 mg of samples at 25 °C. The results indicate that:
The weight gain of Form CS1 under 80%RH is 0.042%. Form CS1 is almost non hygroscopic. The DVS plot of Form CS1 is depicted in FIG. 17. Furthermore, the crystalline form of Form CS1 before and after DVS was tested by XRPD, and the results are displayed in FIG. 18 (at the top is the XRPD pattern before DVS and bottom is the XRPD pattern after DVS). The XRPD overlay indicates that no form change of Form CS1 is observed before and after DVS test.

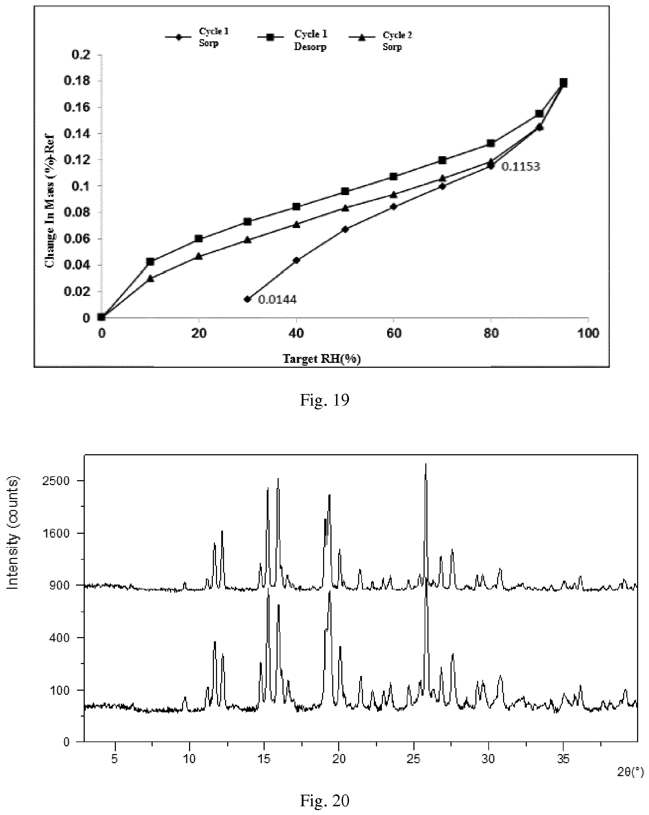

The weight gain of Form CS2 from 30%RH to 80%RH is 0.101%. Form CS2 is almost non hygroscopic and convenient for long-term storage. The DVS plot of Form CS2 is depicted in FIG. 19. Furthermore, the crystalline form before and after DVS was tested by XRPD, and the results are displayed in FIG. 20 (top: XRPD pattern before DVS; bottom: XRPD pattern after DVS). The XRPD overlay indicates that no form change of Form CS2 is observed before and after DVS test.

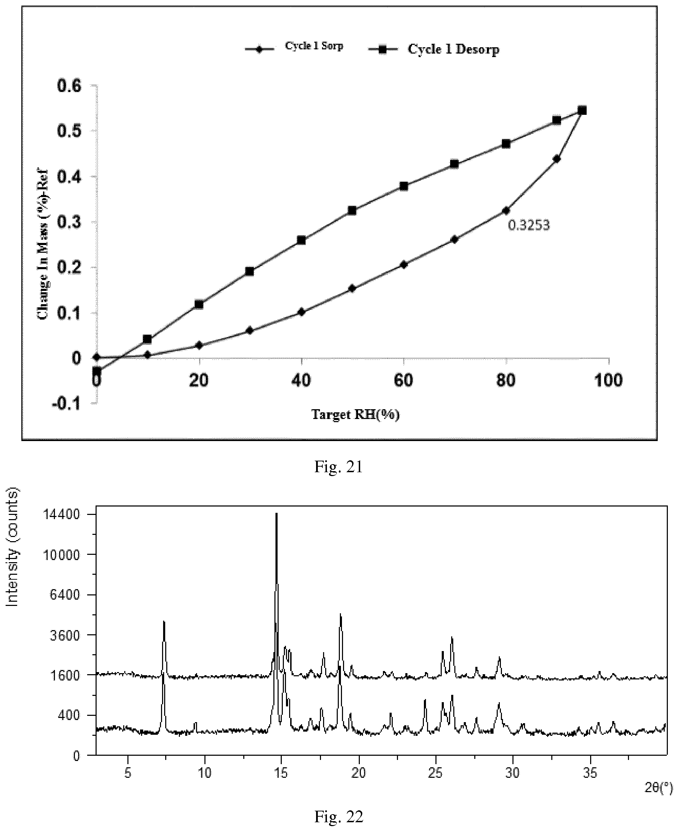

The weight gain of Form CS5 under 80%RH is 0.325%. Form CS5 is slightly hygroscopic. The DVS plot of Form CS5 is depicted in FIG. 21. Furthermore, the crystalline form before and after DVS was tested by XRPD, and the results are displayed in FIG. 22 (top: XRPD pattern before DVS; bottom: XRPD pattern after DVS), and the XRPD overlay indicates that no form change of Form CS5 is observed before and after DVS test.

Description and definition of hygroscopicity (Chinese Pharmacopoeia 2015 edition general principle 9103 Drug hygroscopic test guidelines, test at 25 °C+/-1 °C, 80%RH.).
Deliquescent: Sufficient water is absorbed to form a liquid;
Very hygroscopic: Increase in mass is equal to or greater than 15 percent;
Hygroscopic: Increase in mass is less than 15 percent and equal to or greater than 2 percent;
Slightly hygroscopic: Increase in mass is less than 2 percent and equal to or greater than 0.2 percent.
Non hygroscopic or almost non hygroscopic: Increase in mass is less than 0.2%.

### Example 12: Stability study of Form CS1, Form CS2 and Form CS5

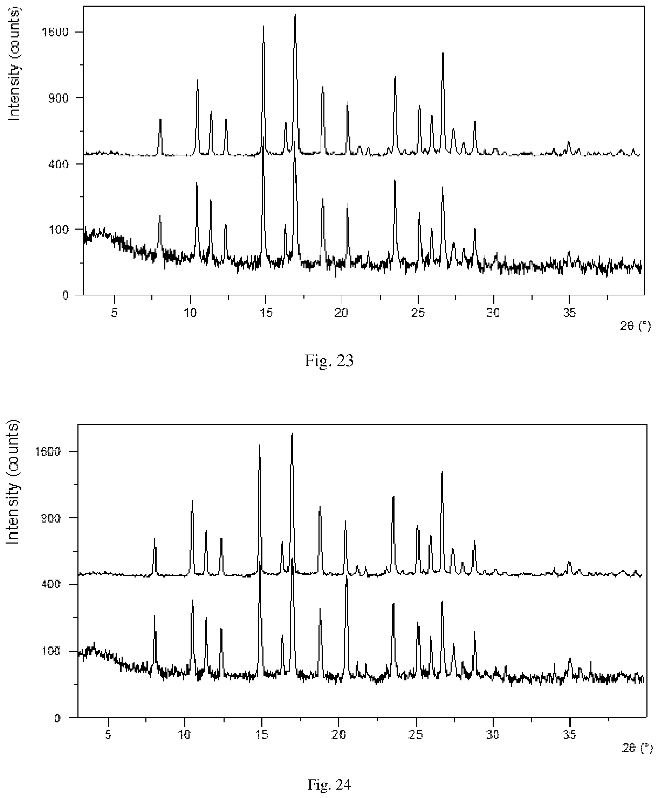

Form CS1 of the present disclosure was stored under different conditions of 25 °C/60%RH, 40 °C/75%RH and 60 °C/75%RH in open dishes. Crystalline form was checked by XRPD. The XRPD overlays of the solid before and after storage are shown in FIG. 23, FIG. 24, and FIG. 25. The stability results are listed in Table 10:

**Table 10**

| Initial solid form | Condition | Time | Crystalline form after storage |
|---|---|---|---|
| Form CS1 | 25 °C/60%RH | 6 months | Form CS1 |
| (top in FIG. 23) | | | (Bottom in FIG. 23) |
| Form CS1 | 40 °C /75%RH | 6 months | Form CS1 |
| (top in FIG. 24) | | | (Bottom in FIG. 24) |
| Form CS1 | 60 °C /75%RH | 1 months | Form CS1 |
| (top in FIG. 25) | | | (Bottom in FIG. 25) |

Form CS1 of the present disclosure is stable for 6 months at 25 °C/60% RH and 40 °C/75% RH. Form CS1 is stable for 1 month at 60 °C/75% RH. It can be seen that Form CS1 of the present disclosure has good stability.

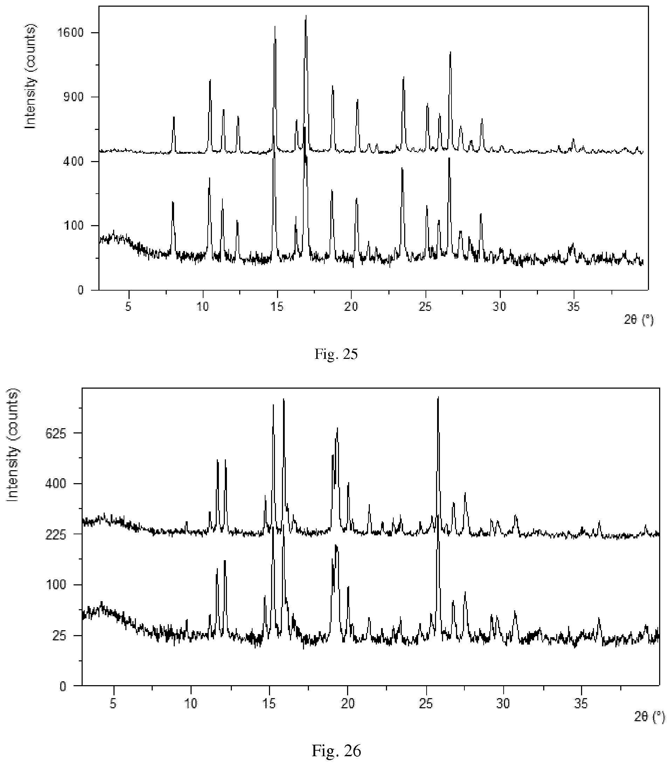

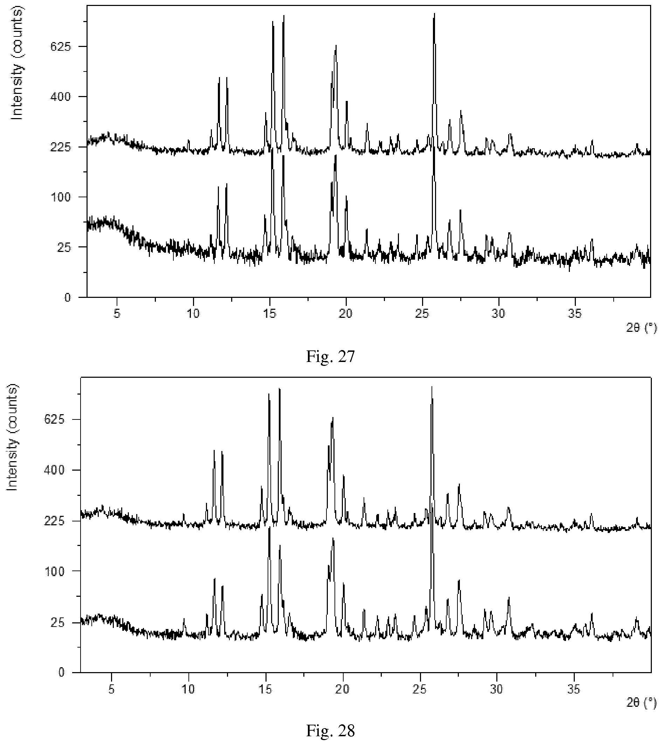

Form CS2 of the present disclosure was stored under different conditions of 25 °C/60%RH, 40 °C/75%RH and 60 °C/75%RH in open dishes. Crystalline form was checked by XRPD. The XRPD overlays of the solid before and after storage are shown in FIG. 26, FIG. 27, and FIG. 28. The stability results are listed in Table 11:

**Table 11**

| Initial solid form | Condition | Time | Crystalline form after storage |
|---|---|---|---|
| Form CS2 | 25 °C/60%RH | 6 months | Form CS2 |
| (Top in FIG. 26) | | | (Bottom in FIG. 26) |
| Form CS2 | 40 °C /75%RH | 6 months | Form CS2 |
| (Top in FIG. 27) | | | (Bottom in FIG. 27) |
| Form CS2 | 60 °C /75%RH | 1 months | Form CS2 |
| (Top in FIG. 28) | | | (Bottom in FIG. 28) |

Form CS2 of the present disclosure is stable for 6 months at 25 °C/60% RH and 40 °C/75% RH. Form CS2 is stable for 1 month at 60 °C/75% RH. It can be seen that Form CS2 of the present disclosure has good stability.

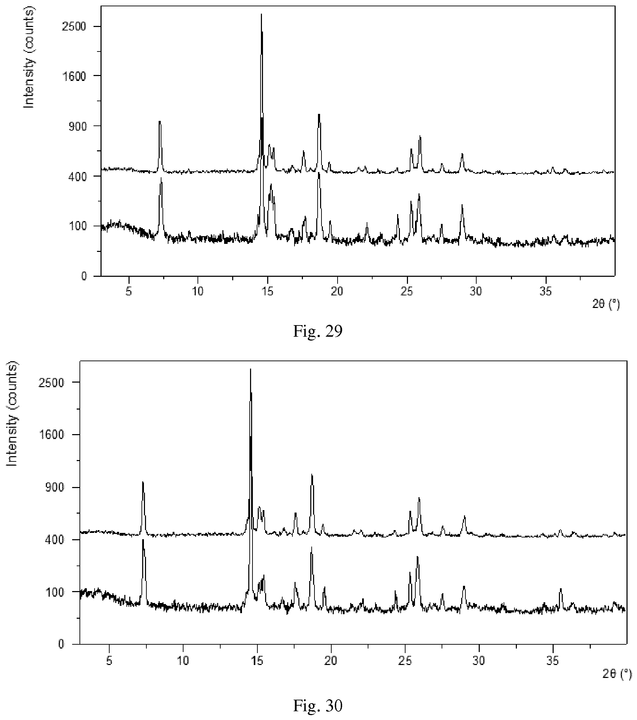

Form CS5 of the present disclosure was stored under different conditions of 25 °C/60%RH, 40 °C/75%RH and 60 °C/75%RH in open dishes. Crystalline form was checked by XRPD. The XRPD overlays of the solid before and after storage are shown in FIG. 29, FIG. 30, and FIG. 31. The stability results are listed in Table 12:

**Table 12**

| Initial solid form | Condition | Time | Crystalline form after storage |
|---|---|---|---|
| Form CS5 | 25 °C/60%RH | 6 months | Form CS5 |
| (Top in FIG. 29) | | | (Bottom in FIG. 29) |
| Form CS5 | 40 °C /75%RH | 6 months | Form CS5 |
| (Top in FIG. 30) | | | (Bottom in FIG. 30) |
| Form CS5 | 60 °C /75%RH | 1 months | Form CS5 |
| (Top in FIG. 31) | | | (Bottom in FIG. 31) |

Form CS5 of the present disclosure is stable for 6 months at 25 °C/60% RH and 40 °C/75% RH. Form CS5 is stable for 1 month at 60 °C/75% RH. It can be seen that Form CS5 of the present disclosure has good stability.

The stability of drug is very important, especially during the shelf life of the drug in market. Good stability could reduce the risk of the crystal transformation which may cause the change of drug dissolution rate and bioavailability, and is of great significance to ensure the efficacy and safety of the drug and prevent the occurrence of adverse drug reactions. Crystalline form with better stability is controllable during the crystallization process and not easy to produce mixed crystal. Meanwhile, during the formulation and storage processes, crystalline form with better stability is hard to convert into other crystal forms. As a result, consistent and controllable of product quality can be ensured, and the dissolution profile will not change with the storage time.

The results indicate that Form CS1, Form CS2 and Form CS5 of the present disclosure have good stability and meet the stringent requirements in the drug application and formulation process.

### Example 13: Dynamic solubility study of Form CS1, Form CS2, Form CS5, and Form CS6

In order to test the solubility of Form CS1, CS2, CS5, and CS6 of the present disclosure, the following experiments have been conducted by the inventors:
Saturated solutions of Form CS1, Form CS2, and Form CS5 of the present disclosure were prepared with SGF (Simulated gastric fluids), pH5.0 FeSSIF (Fed state simulated intestinal fluids), pH6.5 FaSSIF (Fasted state simulated intestinal fluids) and H₂O. After equilibrated for 1 h, 4 h, and 24 h, concentrations of drug substance in the saturated solutions were measured by HPLC. Saturated solutions of Form CS6 of the present disclosure were prepared with SGF (Simulated gastric fluids), pH5.0 FeSSIF (Fed state simulated intestinal fluids) and pH6.5 FaSSIF (Fasted state simulated intestinal fluids). After equilibrated for 1 h, 4 h, and 24 h, concentrations of drug substance in the saturated solutions were measured by HPLC. The solubility results of Form CS1 are listed in Table 13. The solubility results of Form CS2 are listed in Table 14. The solubility results of Form CS5 are listed in Table 15. The solubility results of Form CS6 are listed in Table 16.

**Table 13 Solubility of Form CS1 in SGF, FeSSIF, FaSSIF and H₂O**

| | Time | SGF | FeSSIF | FaSSIF | H₂O |
|---|---|---|---|---|---|
| Solubility (µg/mL) | 1 hour | 7.9 | 122.6 | 417.0 | 4.6 |
| | 4 hours | 5.5 | 141.5 | 428.6 | 10.2 |
| | 24 hours | 3.9 | 139.8 | 420.4 | 4.8 |

**Table 14 Solubility of Form CS2 in SGF, FeSSIF, FaSSIF and H₂O**

| | Time | SGF | FeSSIF | FaSSIF | H₂O |
|---|---|---|---|---|---|
| Solubility (µg/mL) | 1 hour | 0.7 | 30.3 | 35.9 | 3.7 |
| | 4 hours | 1.7 | 31.9 | 42.2 | 15.2 |
| | 24 hours | 1.5 | 27.5 | 37.5 | 37.4 |

**Table 15 Solubility of Form CS5 in SGF, FeSSIF, FaSSIF and H₂O**

| | Time | SGF | FeSSIF | FaSSIF | H₂O |
|---|---|---|---|---|---|
| Solubility (µg/mL) | 1 hour | 14.3 | 86.0 | 187.2 | 0.7 |
| | 4 hours | 24.4 | 123.2 | 300.2 | 16.6 |
| | 24 hours | 42.4 | 114.3 | 321.0 | 42.2 |

**Table 16 Solubility of Form CS6 in SGF, FeSSIF and FaSSIF**

| | Time | SGF | FeSSIF | FaSSIF |
|---|---|---|---|---|
| Solubility (µg/mL) | 1 hour | 1.4 | 34.0 | 6.2 |
| | 4 hours | 6.5 | 84.0 | 29.0 |
| | 24 hours | 17.0 | 180.0 | 27.0 |

Solubility is one of the key properties of drugs, which directly affects the absorption of drugs in the human body. The solubility of different crystalline forms may have obvious difference, and the absorption dynamics in vivo may also change, resulting in differences in bioavailability, which ultimately affects the clinical safety and efficacy of the drug.

The results indicate that the solubility of Form CS1, Form CS2, and Form CS5 of the present disclosure in SGF, FaSSIF, FeSSIF, and H₂O and the solubility of Form CS6 of the present disclosure in SGF, FaSSIF, and FeSSIF meet the medicinal requirements. The solubility in FaSSIF (Fasted state simulated intestinal fluids) and FeSSIF (Fed state simulated intestinal fluids) is relatively high. The favorable solubility of Form CS1, Form CS2, Form CS5, and Form CS6 of the present disclosure can reduce the dose of the drug while ensuring its efficacy, thereby reducing the side effects and improving the safety of the drug. The necessary therapeutic blood concentration after the oral administration can be achieved without high dose, which is beneficial to the absorption of drugs in vivo, achieving ideal bioavailability and efficacy of drugs and meeting the medicinal requirements.

### Example 14: Mechanical stability study of Form CS1, Form CS2, Form CS5, and Form CS6

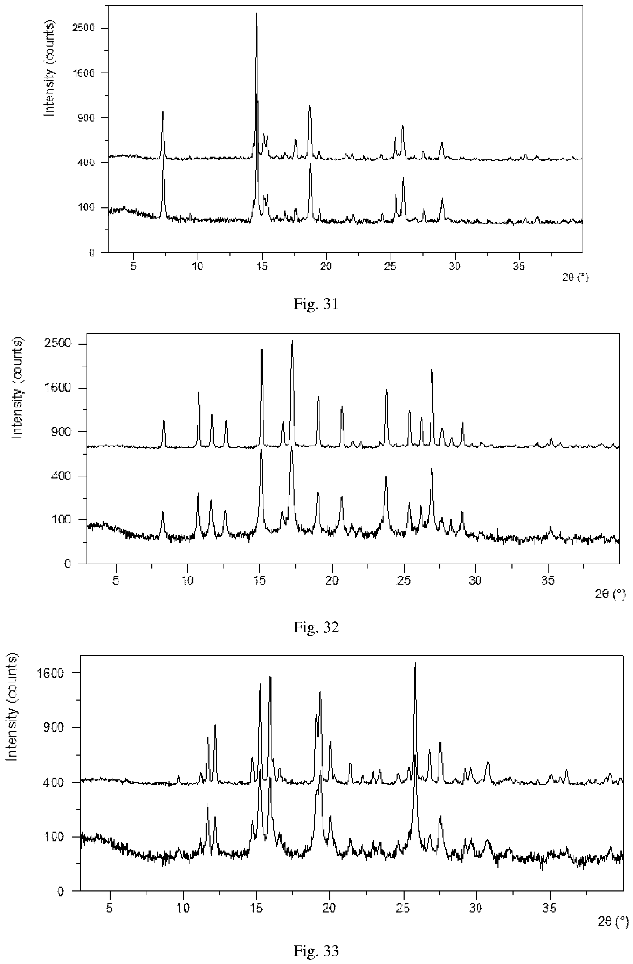

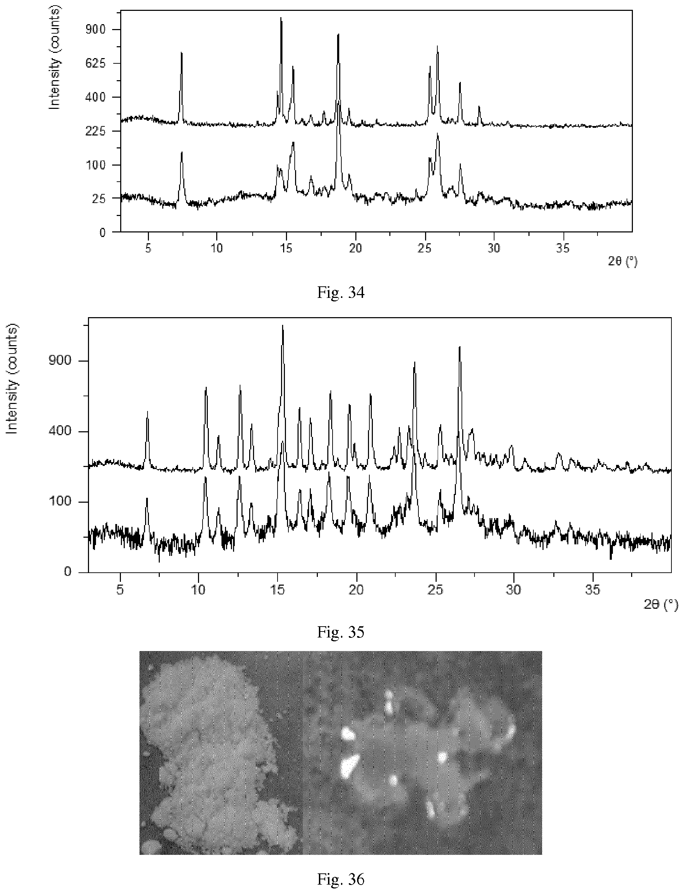

Approximately 20 mg of Form CS1, Form CS2, Form CS5, and Form CS6 of the present disclosure were ground manually with a mortar for 5 minutes, and the solid before and after grinding were tested by XRPD. The results of Form CS1 are displayed in FIG. 32 (top: XRPD pattern before grinding, bottom: XRPD pattern after grinding). The results of Form CS2 are displayed in FIG. 33 (top: XRPD pattern before grinding, bottom: XRPD pattern after grinding). The results of Form CS5 are displayed in FIG. 34 (top: XRPD pattern before grinding, bottom: XRPD pattern after grinding). The results of Form CS6 are displayed in FIG. 35 (top: XRPD pattern before grinding, bottom: XRPD pattern after grinding). As can be seen from the figures, the crystalline forms of Form CS1, Form CS2, Form CS5, and Form CS6 of the present disclosure does not change and the crystallinity is still good.

Crystalline forms with better mechanical stability have good physicochemical properties and remain stable under certain mechanical stress. The crystalline drug with better mechanical stability has low requirements on the crystallization equipment, and no special post-treatment condition is required. It is more stable in the formulation process, can significantly reduce the development cost of the drug products, enhance the quality of the drug, and has strong economic value.

The results indicate that Form CS1, Form CS2, Form CS5, and Form CS6 of the present disclosure have better mechanical stability in the downstream preparation process and provide more choices for the following formulation process. For example, the Form CS1, Form CS2, Form CS5, and Form CS6 can be ground by subsequent dry grinding to obtain drug substance with smaller particle sizes.

### Example 15: morphology comparison among oily substance of prior art, Form CS1, Form CS2, Form CS5, and Form CS6 of the present disclosure

The comparison picture of Form CS1 of the present disclosure and oily substance is displayed in FIG. 36. Form CS1, Form CS2, Form CS5, and Form CS6 are all light yellow powders, which are convenient for sampling and quantification. While the light yellow oily substance prepared according to the prior art is sticky honey-like (right in FIG. 36). The sampling and quantification of oily substance is relatively difficult. Meanwhile, the oily substance has low purity and relatively poor stability, which are not suitable for drug storage. Furthermore, special and complex processing procedures are often needed when the oily substance is used as the active pharmaceutical ingredient to make into solid drug products, which will increase the preparation cost and is difficult for the preparation of formulation.

The examples described above are only for illustrating the technical concepts and features of the present disclosure, and intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. A crystalline form CS1 of compound (I), wherein the X-ray powder diffraction pattern shows characteristic peaks at 2theta values of 10.5°±0.2°, 14.8°±0.2° and 16.9°±0.2°.

2. The crystalline form CS1 according to claim 1, wherein the X-ray powder diffraction pattern shows one or two or three characteristic peaks at 2theta values of 18.7°±0.2°, 20.4°±0.2° and 26.6°±0.2°.

3. The crystalline form CS1 according to claim 1, wherein the X-ray powder diffraction pattern shows one or two or three characteristic peaks at 2theta values of 11.4°±0.2°, 23.5°±0.2° and 25.1°±0.2°.

4. A process for preparing crystalline form CS1 according to any one of claims 1 to 3, wherein the process comprises:
(1) dissolving GFT-505 in a solvent of ketones, adding an anti-solvent, stirring, isolating and drying to obtain the solid; or
(2) dissolving GFT-505 in a solvent mixture comprising aromatic hydrocarbons and ketones at 40-70 °C, precipitating the crystal at 0-10°C, isolating and drying to obtain the solid.

5. The process for preparing crystalline form CS1 according to claim 4, wherein:
in method (1), said ketone includes acetone, methyl ethyl ketone or a solvent mixture of acetone and methyl ethyl ketone, said alkane includes n-hexane, n-heptane, n-octane or a solvent mixture of n-hexane, n-heptane, and n-octane, the volume ratio of said ketone and alkane is 1:20-20:1;
in method (2), said aromatic hydrocarbon includes toluene, ethylbenzene or a solvent mixture of toluene and ethylbenzene, said ketone includes acetone, methyl ethyl ketone, methyl isobutyl ketone or a solvent mixture of acetone, methyl ethyl ketone, and methyl isobutyl ketone, the volume ratio of said aromatic hydrocarbon and ketone is 1:20-20:1, said high temperature is 50 °C and the low temperature is 4 °C.

6. The process for preparing crystalline form CS 1 according to claim 5, wherein:
in method (1), said ketone is acetone, said hydrocarbon is n-heptane, said volume ratio of ketone and hydrocarbon is 1:20;
in method (2), said aromatic hydrocarbon is toluene, said ketone is methyl isobutyl ketone, said volume ratio of aromatic hydrocarbon and ketone is 13:1.

7. A crystalline form CS2 of compound (I), wherein the X-ray powder diffraction pattern shows characteristic peaks at 2theta values of 15.2°±0.2°, 15.9°±0.2° and 25.8°±0.2°.

8. The crystalline form CS2 according to claim 7, wherein the X-ray powder diffraction pattern shows one or two or three characteristic peaks at 2theta values of 11.7°±0.2°, 12.2°±0.2° and 19.4°±0.2°.

9. The crystalline form CS2 according to claim 7, wherein the X-ray powder diffraction pattern shows one or two or three characteristic peaks at 2theta values of 20.0°±0.2°, 26.8°±0.2° and 27.5°±0.2°.

10. A process for preparing crystalline form CS2 according to any one of claims 7 to 9, wherein the process comprises:
(1) suspending GFT-505 in water or a solvent mixture comprising alcohols and water, stirring, isolating and drying to obtain the solid; or
(2) dissolving GFT-505 in a solvent of alcohols, adding water as an anti-solvent, stirring, isolating and drying to obtain the solid; or
(3) dissolving GFT-505 in a solvent mixture comprising alcohols and alkanes, adding polymer and evaporating at 10-50 °C to obtain the solid.

11. The process for preparing crystalline form CS2 according to claim 10, wherein:
in method (1), said alcohol includes methanol, ethanol, isopropanol or a solvent mixture of methanol, ethanol, and isopropanol, said volume ratio of alcohol and water is 1:5-5:1;
in method (2), said alcohol includes methanol, ethanol, isopropanol or a solvent mixture of methanol, ethanol, and isopropanol, said volume ratio of alcohol and water is 1:10-10:1;
in method (3), said alcohol includes methanol, ethanol, isopropanol or a solvent mixture of methanol, ethanol, and isopropanol, said alkane includes hexane, n-heptane, n-octane or a solvent mixture of hexane, n-heptane, and n-octane, said volume ratio of alcohol and alkane is 1:15-15:1, said evaporation temperature is room temperature.

12. The process for preparing crystalline form CS2 according to claim 11, wherein:
in method (1), said alcohol is ethanol, said volume ratio of alcohol and water is 4:5;
in method (2), said alcohol is methanol, aid volume ratio of alcohol and water is 1:7;
in method (3), said alcohol is ethanol, said alkane is n-heptane, said volume ratio of alcohol and alkane is 15:4.

13. A crystalline form CS5 of compound (I), wherein the X-ray powder diffraction pattern shows characteristic peaks at 2theta values of 7.4°±0.2°, 14.6°±0.2° and 18.7°±0.2°.

14. The crystalline form CS5 according to claim 13, wherein the X-ray powder diffraction pattern shows one or two or three characteristic peaks at 2theta values of 25.3°±0.2°, 15.4°±0.2° and 25.9°±0.2°.

15. The crystalline form CS5 according to claim 13, wherein the X-ray powder diffraction pattern shows one or two or three characteristic peaks at 2theta values of 19.5°±0.2°, 27.5°±0.2° and 28.9°±0.2°.

16. A process for preparing crystalline form CS5 according to any one of claims 13 to 15, wherein the process comprises: dissolving GFT-505 in a solvent mixture comprising ketones and aromatic hydrocarbons or a solvent mixture comprising esters and aromatic hydrocarbons, evaporating at 10-50 °C to obtain the solid.

17. The process for preparing crystalline form CS5 according to claim 16, wherein: said ketone includes acetone, methyl ethyl ketone or a solvent mixture of acetone and methyl ethyl ketone, said aromatic hydrocarbon includes toluene, ethylbenzene or a solvent mixture of toluene and ethylbenzene, said ester is ethyl acetate, isopropyl acetate or a solvent mixture of ethyl acetate and isopropyl acetate, said volume ratio of ketones and aromatic hydrocarbons or esters and aromatic hydrocarbons is 1:3-3:1, said evaporation temperature is room temperature.

18. The process for preparing crystalline form CS5 according to claim 17, wherein: said ketone is acetone, said aromatic hydrocarbon is toluene, said ester is ethyl acetate, said volume ratio of ketones and aromatic hydrocarbons or esters and aromatic hydrocarbons is 1:1.

19. A crystalline form CS6 of compound (I), wherein said crystalline form CS6 is an acetic acid solvate, and the X-ray powder diffraction pattern shows characteristic peaks at 2theta values of 12.5°±0.2°, 19.4°±0.2° and 23.6°±0.2°.

20. The crystalline form CS6 according to claim 19, wherein the X-ray powder diffraction pattern shows one or two or three characteristic peaks at 2theta values of 15.2°±0.2°, 20.7°±0.2° and 26.4°±0.2°.

21. The crystalline form CS6 according to claim 19, wherein the X-ray powder diffraction pattern shows one or two or three characteristic peaks at 2theta values of 6.6°±0.2°, 10.3°±0.2° and 18.2°±0.2°.

22. A process for preparing crystalline form CS6 according to any one of claims 19 to 21, wherein the process comprises: placing GFT-505 in a closed container which contains solvent atmosphere of acetic acid to obtain the solid by solid vapor diffusion.

23. A pharmaceutical composition, wherein said pharmaceutical composition comprises a therapeutically effective amount of crystalline form CS1 according to any one of claims 1 to 3, crystalline form CS2 according to any one of claims 7 to 9, crystalline form CS5 according to any one of claims 13 to 15, or crystalline form CS6 according to any one of claims 19 to 21 and pharmaceutically acceptable carriers, diluents or excipients.

24. Crystalline form CS1 according to any one of claims 1 to 3, crystalline form CS2 according to any one of claims 7 to 9, crystalline form CS5 according to any one of claims 13 to 15, or crystalline form CS6 according to any one of claims 19 to 21 for the use of preparing drug products treating nonalcoholic steatohepatitis and/or type 2 diabetes and/or dyslipidemia and/or atherosclerosis.
